Europäisches Patentamt

European Patent Office

Office européen des brevets

(11) Numéro de publication : **0 185 575**
**B1**

# (12) FASCICULE DE BREVET EUROPÉEN

(45) Date de publication du fascicule du brevet :
**28.09.88**

(21) Numéro de dépôt : **85402274.6**

(22) Date de dépôt : **22.11.85**

(51) Int. Cl.⁴ : **A 61 M 16/00**

(54) **Procédé et dispositif de réchauffage de gaz.**

(30) Priorité : **23.11.84 FR 8417850**

(43) Date de publication de la demande :
**25.06.86 Bulletin 86/26**

(45) Mention de la délivrance du brevet :
**28.09.88 Bulletin 88/39**

(84) Etats contractants désignés :
**AT BE CH DE GB IT LI LU NL SE**

(56) Documents cités :
**FR-A- 2 363 336**
**GB-A- 2 011 792**

(73) Titulaire : **DUFFOUR ET IGON S.A. (D.I.)**
**Rue de l'Oasis B.P. 3084**
**F-31025 Toulouse Cedex (FR)**

(72) Inventeur : **Vigneau, Jean-Louis**
**Montegut Plantaurel**
**F-09120 Varilhes (FR)**
Inventeur : **Trenque, Pierre**
**1, Résidence le Parc Avenue des Croisés**
**F-31520 Ramonville St Agne (FR)**

(74) Mandataire : **Chameroy, Claude et al**
**c/o Cabinet Maiemont 42, avenue du Président Wilson**
**F-75116 Paris (FR)**

## Description

La présente invention concerne un procédé de réchauffage de gaz dans lequel on fait passer simultanément sur une charge de chaux sodée, le gaz à réchauffer et du gaz carbonique provoquant une réaction exothermique avec la chaux sodée. Elle a trait également à un dispositif pour la mise en œuvre de ce procédé.

Le réchauffage d'un gaz initialement froid ou à la température ambiante est notamment utilisé dans le domaine médical. Dans cette application particulière, on réchauffe de l'air ou de l'oxygène, que l'on fait inhaler à un patient afin de lui apporter des calories supplémentaires. On peut ainsi lutter efficacement contre l'hypothermie, que celle-ci soit accidentelle, comme dans le cas des accidentés de la route, de la montagne ou les noyés, ou occasionnée par exemple par une opération chirurgicale de longue durée ou un séjour prolongé à basse température.

A cet effet, on a déjà pensé à utiliser la réaction exothermique de la chaux sodée en présence de gaz carbonique, ce qui a pour avantage de rendre la production de calories indépendante d'une source d'énergie extérieure. Cette réaction, dont la formule est la suivante :
$$2CaO + 2NaOH + 3CO_2 \rightarrow 2CaCO_3 + N_2CO_3 + H_2O + \text{chaleur},$$
est très connue et elle est d'ailleurs déjà utilisée dans le domaine médical pour épurer les gaz expirés par les patients en cours d'anesthésie.

GB-A-2 011 792 décrit un dispositif dans lequel l'air inhalé passe par une cartouche contenant de la chaux sodée. La réaction du gaz carbonique contenu dans l'air avec la chaux sodée permet de réchauffer l'air inhalé. La réaction est amorcée en faisant passer durant 10 à 15 secondes de l'air comprimé ou en expirant dans la cartouche, cependant ce dispositif présente des difficultés à maintenir la réaction. En effet, la température en début de réaction peut monter au-dessus du seuil admissible par le patient. De plus, le gaz carbonique saturant la chaux sodée, lorsque le seuil de saturation est dépassé, il y a un risque d'inhalation par le patient d'une concentration de gaz carbonique inacceptable. Enfin, la production massive de calories en début de réaction provoque une surconsommation qui est préjudiciable à l'autonomie du système.

La présente invention a pour but principal de remédier à ces inconvénients et, pour ce faire, elle a pour objet un procédé du type susmentionné qui se caractérise essentiellement en ce qu'il consiste à mettre la chaux sodée en présence d'une quantité prédéterminée de gaz carbonique afin d'amorcer rapidement la réaction exothermique et à faire passer ensuite sur la chaux sodée un mélange de gaz à réchauffer et de gaz carbonique en proportion déterminée afin d'entretenir ladite réaction exothermique.

On arrive ainsi à contrôler parfaitement la réaction, ce qui permet notamment d'obtenir un réchauffage puissant, rapide et stable pendant une durée relativement longue. De plus, la température de réchauffage peut être réglée facilement en faisant simplement varier la proportion du mélange de gaz à réchauffer et de gaz carbonique.

Le dispositif pour la mise en œuvre du procédé selon l'invention est essentiellement caractérisé en ce qu'il comprend une cartouche avec une première chambre renfermant la charge de chaux sodée et une seconde chambre renfermant la quantité de gaz carbonique nécessaire à l'amorçage de la réaction exothermique, et un moyen pour mettre les deux chambres en communication, la première chambre étant reliée d'une part à une entrée pour le mélange de gaz à réchauffer et de gaz carbonique de maintien de la réaction, et d'autre part à une sortie pour le gaz réchauffé.

De préférence, le dispositif comprend également un corps de réchauffeur sur lequel la cartouche vient se fixer de manière amovible, la mise en place de la cartouche sur le corps de réchauffeur provoquant automatiquement la mise en communication des deux chambres.

On dispose ainsi d'un ensemble extrêmement pratique et qui peut être mis en œuvre très rapidement dans les cas d'urgence.

Dans une forme de réalisation particulière de l'invention, la première chambre est ménagée au centre de la cartouche, tandis que la seconde chambre est constituée par une chambre annulaire hermétique entourant la première chambre, ladite première chambre centrale débouchant à sa partie supérieure dans une chambre de décantation reliée à la sortie de gaz réchauffé et à sa partie inférieure dans une chambre d'admission ménagée entre le fond de la cartouche et l'embase du corps de réchauffeur sur lequel est disposée l'entrée pour le mélange de gaz à réchauffer et de gaz carbonique de maintien.

Par ailleurs, la première chambre centrale est avantageusement séparée de la chambre d'admission par une grille assurant une répartition uniforme du flux gazeux sur la chaux sodée et de la chambre de décantation par un filtre à poussières.

De préférence, le moyen provoquant automatiquement la mise en communication des deux chambres est constitué par un percuteur placé sur le corps de réchauffeur et apte à perforer une membrane obturant hermétiquement la seconde chambre lors de la mise en place de la cartouche sur le corps de réchauffeur, le gaz carbonique d'amorçage s'écoulant alors vers la première chambre par l'intermédiaire de la chambre d'admission.

Une forme d'exécution de l'invention est décrite ci-après à titre d'exemple, en référence au dessin annexé dans lequel :

— la figure 1 est une vue simplifiée d'un dispositif de réchauffage de gaz conforme à l'invention ; et

— la figure 2 est une vue en coupe à plus grande échelle suivant la ligne II-II de la figure 1.

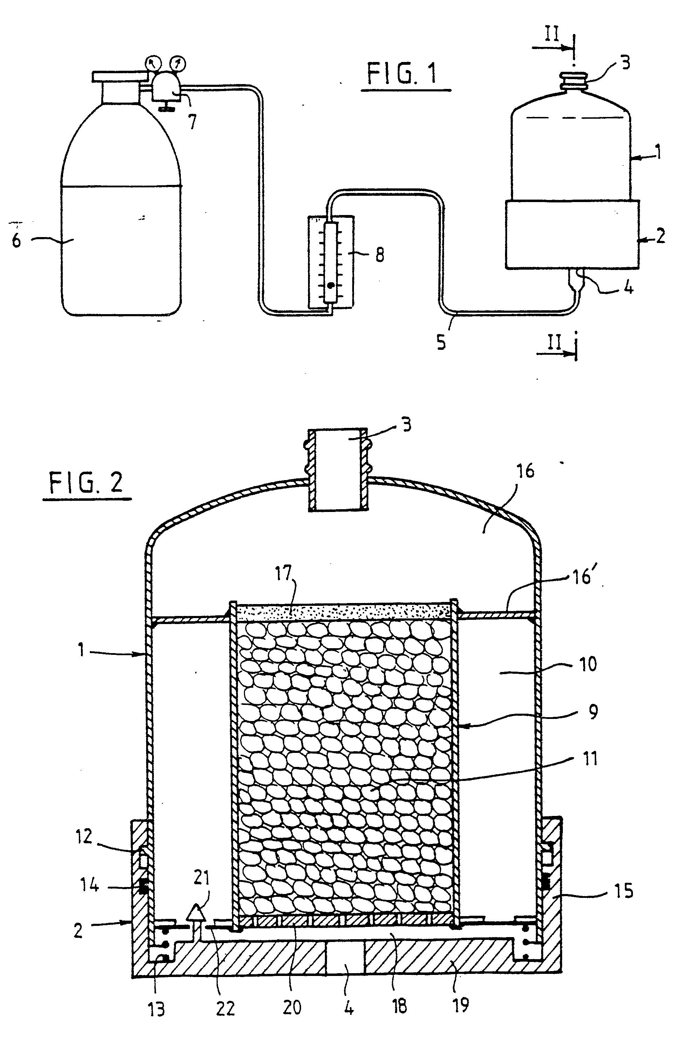

Le dispositif représenté sur la figure 1 comprend tout d'abord une cartouche 1 venant se fixer de manière amovible sur un corps de réchauffeur 2. La cartouche 1 est surmontée d'un embout 3 constituant une sortie pour le gaz réchauffé, tandis que le corps de réchauffeur 2 comporte à sa partie inférieure une entrée 4 qui est reliée par une tuyauterie 5 à une bouteille 6 contenant un mélange comprimé de gaz froid à réchauffer et de gaz carbonique. Un détendeur-régulateur 7 est disposé à la sortie de la bouteille 6 et un générateur de débit constant 8 est par ailleurs interposé sur la tuyauterie 5, de manière que le mélange de gaz arrive au corps de réchauffeur 2 avec un débit et une pression convenables.

Ainsi qu'on peut le voir plus clairement sur la figure 2, la cartouche 1 est essentiellement constituée par une enceinte de forme cylindrique à l'intérieur de laquelle sont ménagées une première chambre centrale 9 et une seconde chambre annulaire 10 entourant la première. La chambre centrale 9 contient une quantité prédéterminée de chaux sodée 11, tandis que la chambre annulaire 10 contient une quantité prédéterminée de gaz carbonique nécessaire à l'amorçage de la réaction exothermique avec la chaux sodée 11.

La cartouche 1 est fixée de manière amovible sur le corps de réchauffeur 2 par un moyen approprié, par exemple par vissage, ou, comme représenté en 12, par un système à baïonnette fonctionnant en association avec un ressort de compensation 13 venant se loger dans le corps de réchauffeur. Un joint torique 14 est en outre prévu sur la partie cylindrique 15 du corps de réchauffeur 2 pour assurer une liaison étanche avec la cartouche.

La chambre centrale 9 contenant la chaux sodée 11 débouche à sa partie supérieure dans une chambre de décantation 16 reliée à la sortie 3 de gaz réchauffé et qui est destinée à recueillir l'eau qui se condense sur le haut de la cartouche. Le cas échéant, on pourra rajouter un condenseur pour les applications où la valeur d'eau engendrée par la réaction est indésirable. Un filtre à poussières 17 est en outre interposé entre la chaux sodée 11 et la chambre de décantation 16.

On notera par ailleurs que la paroi constituant la chambre centrale 9 déborde à l'intérieur de la chambre de décantation 16, formant ainsi une zone de retenue annulaire 16' dans laquelle s'accumule l'eau de condensation. Il est en effet important que cette eau ne vienne pas en contact avec la chaux sodée 11. Le cas échéant, on pourra disposer dans la zone annulaire 16' un matériau absorbant, afin de pouvoir utiliser la cartouche dans une position plus ou moins inclinée.

A sa partie inférieure, la chambre centrale 9 débouche dans une chambre d'admission 18 ménagée entre le fond de la cartouche 1 et l'embase 19 du corps de réchauffeur 2, chambre d'admission dans laquelle débouche également l'entrée 4 pour le mélange de gaz à réchauffer et de gaz carbonique. Une grille 20 est par ailleurs interposée entre la chambre centrale 9 et la chambre d'admission 18 pour assurer une répartition uniforme du flux gazeux sur la masse de chaux sodée 11.

Sur l'embase 19 du corps de réchauffeur 2 est également disposé latéralement un percuteur 21 apte à perforer automatiquement, lors de la mise en place de la cartouche 1, une membrane 22, par exemple en clinquant, obturant normalement de manière hermétique la chambre annulaire 10 dans laquelle se trouve le gaz carbonique nécessaire à l'amorçage de la réaction.

La cartouche 1 est normalement conçue pour être à usage unique. En effet, comme le gaz carbonique nécessaire à l'amorçage de la réaction aura été libéré totalement lors de la première utilisation par l'action du percuteur 21, la cartouche ne pourra resservir une deuxième fois, même si elle n'a été utilisée que partiellement.

Dans cette optique, la cartouche pourra être conçue, soit pour être jetée après usage, soit pour être reconditionnée industriellement. Dans ce dernier cas, des moyens devront être prévus pour permettre le remplacement de la charge de chaux sodée 11 et le remplissage de la chambre annulaire 10 avec une nouvelle quantité de gaz carbonique d'amorçage.

Pour éviter l'altération de la chaux sodée 11, la cartouche 1 sera obturée à son entrée, c'est-à-dire au niveau de la grille 20, et à sa sortie 3, par un bouchon ou un film étanche qu'il conviendra d'arracher lors de la mise en service. Cette disposition permettra également de distinguer aisément une cartouche neuve d'une cartouche usagée.

Après avoir raccordé la sortie 3 au circuit d'utilisation, constitué par exemple par un inhalateur dans le cas du traitement de l'hypothermie, on introduit la cartouche 1 dans le corps de réchauffeur 2, par l'intermédiaire du système à baïonnette 12 et on la verrouille en position. Ce faisant, le percuteur 21 perfore le clinquant 22 en réalisant un orifice calibré par lequel s'échappe le gaz carbonique d'amorçage contenu à l'intérieur de la chambre annulaire 10. Comme une étanchéité est réalisée au niveau du joint 14, ce gaz carbonique se répand d'abord dans la chambre d'admission 18 et pénètre ensuite dans la chambre centrale 9 contenant la charge de chaux sodée, par l'intermédiaire de la grille diviseur de flux 20.

La réaction exothermique s'amorce alors immédiatement. La température s'élève en quelques secondes et atteint le niveau requis en deux à trois minutes environ.

Il ne reste plus ensuite qu'à injecter au niveau de l'entrée 4 le gaz froid à réchauffer et le gaz carbonique nécessaire à l'entretien de la réaction exothermique. Dans le mode de réalisation particulier représenté ici, le gaz carbonique de maintien est préalablement dilué dans le gaz froid, formant ainsi un mélange prédéterminé. On a alors une seule source, constituée par la bouteille 6, ce qui est particulièrement commode dans les cas d'urgence, par exemple pour le traitement d'accidentés, et permet une réalisation très compacte facilitant le transport.

Il va de soi cependant que l'on pourrait également utiliser des alimentations séparées en gaz froid et en gaz carbonique de maintien, et réaliser le mélange sur place, soit directement au niveau de la chambre d'admission 18 avec des entrées distinctes pour les deux gaz, soit en amont avec une seule entrée sur le corps de réchauffeur.

Il va de soi en outre que le gaz carbonique d'amorçage et le mélange de gaz froid et de gaz carbonique de maintien peuvent être injectés dans la cartouche successivement, ou bien simultanément, sans modifier sensiblement les caractéristiques. On pourrait ainsi en particulier alimenter l'entrée 4 avec le mélange de gaz froid et de gaz carbonique de maintien avant de mettre la cartouche 1 en place sur le corps de réchauffeur 2.

Pour des raisons de sécurité, il faut que la masse totale de gaz carbonique d'amorçage et de gaz carbonique de maintien soit inférieure à la masse de saturation de la charge de chaux sodée. A titre d'exemple, avec une masse de chaux sodée de 0,7 kg, on pourra prendre une bouteille standard de cinq litres de capacité en eau, capable de contenir 1 m$^3$ de gaz comprimé à 200.10$^5$ Pa, avec une concentration de 7,5 % en gaz carbonique de maintien, la masse de gaz carbonique d'amorçage étant alors d'environ 25 grammes.

Le détendeur-régulateur 7 ramène la pression de stockage du gaz comprimé à une valeur compatible avec l'utilisation, soit environ 1 000 à 3 000 Pa, tandis que le générateur de débit 8 calibre le débit de soutirage à une valeur constante fonction de la température de réchauffage souhaitée. On obtient ainsi des températures comprises entre 40 et 80 °C pour des débits de gaz carbonique de maintien allant de 0,5 à 1,5 l/mn.

Avec des alimentations séparées en gaz froid et en gaz carbonique de maintien, il est bien entendu plus facile de faire varier la température de réchauffage, car on peut alors faire varier la concentration en gaz carbonique sans modifier sensiblement le débit à la sortie.

Il va de soi par ailleurs que le dispositif de réchauffage selon l'invention pourrait également être équipé de diverses sécurités complémentaires. Ainsi par exemple, on pourrait prévoir une sonde de température pour mesurer la température du gaz chaud à la sortie de la cartouche 1. Dans ce cas, la sonde serait placée le plus près possible de l'utilisation, pour tenir compte des pertes thermiques en ligne.

On peut aussi, le cas échéant, prévoir un petit analyseur de gaz carbonique, afin de s'assurer que le gaz chaud sortant de la cartouche ne contient pas de gaz carbonique.

**Revendications**

1. Procédé de réchauffage de gaz dans lequel on fait passer simultanément sur une charge de chaux sodée, le gaz à réchauffer et du gaz carbonique provoquant une réaction exothermique avec la chaux sodée, caractérisé en ce qu'il consiste à mettre la chaux sodée en présence d'une quantité prédéterminée de gaz carbonique afin d'amorcer rapidement la réaction exothermique et à faire passer ensuite sur la chaux sodée un mélange de gaz à réchauffer et de gaz carbonique en proportion déterminée afin d'entretenir ladite réaction exothermique.

2. Dispositif pour la mise en œuvre du procédé selon la revendication 1, caractérisé en ce qu'il comprend une cartouche (1) avec une première chambre (9) renfermant la charge de chaux sodée (11) et une seconde chambre (10) renfermant la quantité de gaz carbonique nécessaire à l'amorçage de la réaction exothermique, et un moyen pour mettre les deux chambres en communication, la première chambre (9) étant reliée d'une part à une entrée (4) pour le mélange de gaz à réchauffer et de gaz carbonique de maintien de la réaction, et d'autre part à une sortie (3) pour le gaz réchauffé.

3. Dispositif selon la revendication 2, caractérisé en ce qu'il comprend également un corps de réchauffeur (2) sur lequel la cartouche (1) vient se fixer de manière amovible, la mise en place de la cartouche sur le corps de réchauffeur provoquant automatiquement la mise en communication des deux chambres (9, 10).

4. Dispositif selon la revendication 3, caractérisé en ce que la première chambre (9) est ménagée au centre de la cartouche (1), tandis que la seconde chambre (10) est constituée par une chambre annulaire hermétique entourant la première chambre, ladite première chambre centrale (9) débouchant à sa partie supérieure dans une chambre de décantation (16) reliée à la sortie (3) de gaz réchauffé et à sa partie inférieure dans une chambre d'admission (18) ménagée entre le fond de la cartouche (1) et l'embase (19) du corps de réchauffeur (2) sur lequel est disposée l'entrée (4) pour le mélange de gaz à réchauffer et de gaz carbonique de maintien.

5. Dispositif selon la revendication 4, caractérisé en ce que la première chambre centrale (9) est séparée de la chambre d'admission (18) par une grille (20) assurant une répartition uniforme du flux gazeux sur la chaux sodée (11) et de la chambre de décantation (16) par un filtre à poussières (17).

6. Dispositif selon la revendication 4 ou 5, caractérisé en ce que le moyen provoquant automatiquement la mise en communication des deux chambres (9, 10) est constitué par un percuteur (21) placé sur le corps de réchauffeur (2) et apte à perforer une membrane (22) obturant hermétiquement la seconde chambre (10) lors de la mise en place de la cartouche (1) sur le corps de réchauffeur (2), le gaz carbonique d'amorçage s'écoulant alors vers la première chambre (9) par l'intermédiaire de la chambre d'admission (18).

**Claims**

1. Gas heating process in which the gas to be heated and carbonic gas are caused to pass simultaneously over a charge of soda-lime with which said carbonic gas causes an exothermic reaction, characterized in that it consists in placing the soda-lime in the presence of a predetermined amount of carbonic gas for rapidly initiating the exothermic reaction and then in causing a mixture of gas to be heated and carbonic gas in given proportion to pass over the soda-lime so as to maintain said exothermic reaction.

2. Device for implementing the process according to claim 1, characterized in that it comprises a cartridge (1) with a first chamber (9) containing the charge of soda-lime (11) and a second chamber (10) containing the amount of carbonic gas required for initiating the exothermic reaction, and a means for placing the two chambers in communication, the first chamber (9) being connected on the one hand to an inlet (4) for the mixture of gas to be heated and of carbonic gas for maintaining the reaction, and on the other hand to an outlet (3) for the heated gas.

3. Device according to claim 2, characterized in that it also comprises a heater body (2) on which the cartridge (1) is removably fixed, positioning of the cartridge on the heater body automatically causing the two chambers (9, 10) to be placed in communication.

4. Device according to claim 3, characterized in that the first chamber (9) is formed in the center of the cartridge (1), whereas the second chamber (10) is formed by an annular hermetic chamber surrounding first chamber, said first central chamber (9) opening at its upper part into a decantation chamber (16) connected to the heated gas outlet (3) and at its lower part into an intake chamber (18) formed between the bottom of the cartridge (1) and the base (19) of the heater body (2) in which is provided the inlet (4) for the mixture of gas to be heated and of maintaining carbonic gas.

5. Device according to claim 4, characterized in that the first central chamber (9) is separated from the intake chamber (18) by a grid (20) ensuring uniform distribution of the gas flow over the soda-lime (11) and from the decantation chamber (16) by a dust filter (17).

6. Device according to claim 4 or 5, characterized in that the means for automatically causing the two chambers (9, 10) to be placed in communication is formed by a striker (21) placed on the heater body (2) and adapted to perforate a membrane (22) hermetically sealing the second chamber (10) during positioning of the cartridge (1) on the heater body (2), the initiating carbonic gas then flowing towards the first chamber (9) through the intake chamber (18).

**Patentansprüche**

1. Verfahren um Gas zu erwärmen, in welchem auf einer Füllung sodahaltigem Kalk das zu erwärmende Gas und Kohlendioxyd gleichzeitig geführt werden, um eine exothermische Reaktion mit dem sodahaltigen Kalk zu bewirken, dadurch gekennzeichnet, daß es daran besteht, den sodahaltigen Kalk mit einer vorherbestimmten Menge Kohlendioxyd zusammenzubringen, um die exothermische Reaktion rasch einzuleiten und danach auf den sodahaltigen Kalk eine Mischung aus dem zu erwärmenden Gas und dem Kohlendioxyd in bestimmter Menge zu führen, um somit die gesagte exothermische Reaktion einzuleiten.

2. Einrichtung zum Einleiten des Verfahrens entsprechend Anspruch 1, dadurch gekennzeichnet, daß sie aus einer Kartusche (1) besteht mit einer ersten Kammer (9), die den sodahaltigen Kalk (11) enthält, und aus einer zweiten Kammer (10) besteht, die die zur Einleitung der exothermischen Reaktion erforderliche Menge Kohlendioxyd enthält, und aus einem Mittel besteht, um beide Kammern kommunizieren zu lassen, wobei die erste Kammer (9) einerseits mit einem Einlaß für die Mischung aus dem zu erwärmenden Gas und Kohlendioxyd zur Erhaltung der Reaktion und andererseits mit einem Auslaß (3) für das erwärmte Gas verbunden ist.

3. Einrichtung entsprechend Anspruch 2, dadurch gekennzeichnet, daß sie ebenfalls einen Aufwärmkörper enthält auf welchen die Kartusche (1) abnehmbar befestigt wird, wobei das Einsetzen der Kartusche auf den Aufwärmkörper die Verbindung beider Kammern (9, 10) automatisch bewirkt.

4. Einrichtung entsprechend Anspruch 3, dadurch gekennzeichnet, daß die erste Kammer (9) in der Mitte der Kartusche (1) eingerichtet ist, während die zweite Kammer (10) aus einer ringförmigen die erste Kammer hermetisch umgebenden Kammer besteht, wobei die gesagte mittige Kammer (9) an ihrem Oberteil in eine Klärkammer (16) mündet, die mit dem Auslaß (3) des erwärmten Gases verbunden ist, und an ihrem Unterteil in eine zwischen dem Boden der Kartusche (1) und der Grundplatte (19) des Aufwärmkörpers (19) eingerichtete Einlaßkammer (18) mündet, wobei der Einlaß (4) für die Mischung aus dem zu erwärmenden Gas und dem für die Selbsthaltung vorgesehenen Kohlendioxyd in dieser Grundplatte eingerichtet ist.

5. Einrichtung entsprechend Anspruch 4, dadurch gekennzeichnet, daß die erste mittige Kammer (9) durch ein Gitter (20), das eine gleichmäßige Aufteilung des Gasstromes auf den sodahaltigen Kalk sichert, von der Einlaßkammer und durch einen Staubfilter (17) von der Klärkammer getrennt ist.

6. Einrichtung entsprechend Anspruch 4 oder 5, dadurch gekennzeichnet, daß das Mittel zur automatischen Verbindung beider Kammern (9, 10) aus einem am Körper des Aufwärmers (2) angebrachten Dorn (21) besteht, der in der Lage ist, die die zweite Kammer hermetisch abschließende Membrane während des Aufsetzens der Kartusche (1) auf den Körper des Aufwärmers (2) durchzustoßen, wobei das Kohlendioxyd dann zur ersten Kammer (9) über die Einlaßkammer (18) strömt.

0 185 575

FIG. 1

FIG. 2